# EUROPEAN PATENT SPECIFICATION

(11) **EP 1 814 538 B1**
(45) Date of publication and mention of the grant of the patent: **23.09.2009**
(21) Application number: 05820776.2
(22) Date of filing: 31.10.2005
(51) Int. Cl.: A61K 31/335

(54) **Method of delivering nasal spray**
Verfahren zur Verabreichung eines Nasensprays
Procédé d'administration d'une pulvérisation nasale

(30) Priority: 24.11.2004 US 630886 P
(43) Date of publication of application: 08.08.2007
(73) Proprietor: Alcon, Inc., 6331 Hünenberg (CH)
(72) Inventor: CAGLE, Gerald, Fort Worth, Texas 76116 (US); WALL, G., Michael, Fort Worth, TX 76109 (US)
(74) Representative: Moore, Barry
(86) International application number: PCT/US2005/039268
(87) International publication number: WO 2006/057769

(56) References cited:
- EP-A1- 1 426 059
- US-A- 4 871 865
- US-A- 6 143 329
- US-A1- 2003 055 102
- US-A1- 2004 034 062

## Description

This application claims the priority of U.S. Provisional Application No. 60/630,886 filed November 24, 2004.

### Field of the Invention

The present invention generally pertains to the delivery of nasal sprays and more particularly to the delivery of nasal sprays containing olopatadine.

### Description of the Related Art

A variety of nasal sprays are available for treating allergic rhinitis. Exemplary products include FLONASE® nasal spray available from GlaxoSmithKline of the United Kingdom; NASONEX® nasal spray available from Schering Corporation of Kennilworth, New Jersey; and ASTELIN® nasal spray available from MedPointe Pharmaceuticals of Somerset, New Jersey. All of these products deliver topical formulations via conventional pump-sprayers available from suppliers such as Pfeiffer of Germany; Saint-Gobain Calmar of France, or Valois of France.

U.S. Patent Nos. 4,871,865 and 4,923,892, both assigned to Burroughs Wellcome Co. ("the Burroughs Wellcome Patents"), disclose that certain carboxylic acid derivatives of doxepin, including olopatadine (chemical name: Z-11-(3-dimethylaminopropylidene)-6,11-dihydrodibenz[b,e]oxepine-2-acetic acid), have antihistamine and antiasthmatic activity. The Burroughs Wellcome Patents teach various pharmaceutical formulations containing the carboxylic acid derivatives of doxepin, including nasal spray and ophthalmic formulations. See, for example, Col. 7, lines 7 - 26, and Examples 8 (H) and 8 (I) of the '865 patent.

U.S. Patent No. 5,116,863, assigned to Kyowa Hakko Kogyo Co., Ltd., ("the Kyowa patent"), teaches that acetic acid derivatives of doxepin and, in particular, olopatadine, have anti-allergic and anti-inflammatory activity. Medicament forms taught by the Kyowa patent for the acetic acid derivatives of doxepin include a wide range of acceptable carriers; however, only oral and injection administration forms are mentioned.

U.S. Patent No. 5,641,805, assigned to Alcon Laboratories, Inc. and Kyowa Hakko Kogyo Co., Ltd., teaches topical ophthalmic formulations containing olopatadine for treating allergic eye diseases. According to the '805 patent, the topical formulations may be solutions, suspensions or gels.

PATANOL^{®} (olopatadine hydrochloride ophthalmic solution) 0.1%, from Alcon Laboratories, Inc. of Fort Worth, Texas, is currently the only commercially available olopatadine product for ophthalmic use. According to its labeling information, it contains olopatadine hydrochloride equivalent to 0.1 % olopatadine, 0.01 % benzalkonium chloride, and unspecified amounts of sodium chloride, dibasic sodium phosphate, hydrochloric acid and/or sodium hydroxide (to adjust pH) and purified water.

U.S. Patent Application Publication No. 20030055102 of Alcon, Inc. discloses topical olopatadine formulations that are effective for treating and/or preventing allergic or inflammatory disorders of the eye or nose. Formulations of aqueous solutions that comprise approximately 0.2 - 0.6% olopatadine are disclosed.

U.S. Patent Nos. 4,871,865; 4,923,892; 5,116,863; and 5,641,805 and U.S. Patent Application Publication No. 20030055102.

Improved methods of delivering topical olopatadine formulations that are effective for treating allergic or inflammatory conditions of the nose remain desirable. Summary of the Invention

Accordingly the invention provides a nasal sprayer according to claim 1. Advantages embodiments are provided in the dependent claims such a sprayer may be used in a method of delivering a nasal spray. A sprayer having a formulation comprising olopatadine is provided. A spray of the formulation is delivered to a subject's nose. The spray has a spray characteristic comprising a spray pattern having a longest axis of 20 - 45 mm, a shortest axis of 14 - 20 mm, and an ellipticity of 1 -1.8, and/or

a spray characteristic comprising a droplet size distribution having a D₁₀ of 15 - 30 µm, a D₅₀ of 30 - 60 µm, a D₉₀ of 50 - 150 µm, a SPAN of not more than 3, and a % Volume of < 10 µm of less than 4% use of olopatadine as provided in claim 10 is also provided.

### Brief Description of the Drawings

For a more complete understanding of the present invention, and for further objects and advantages thereof, reference is made to the following description taken in conjunction with the accompanying drawings in which:

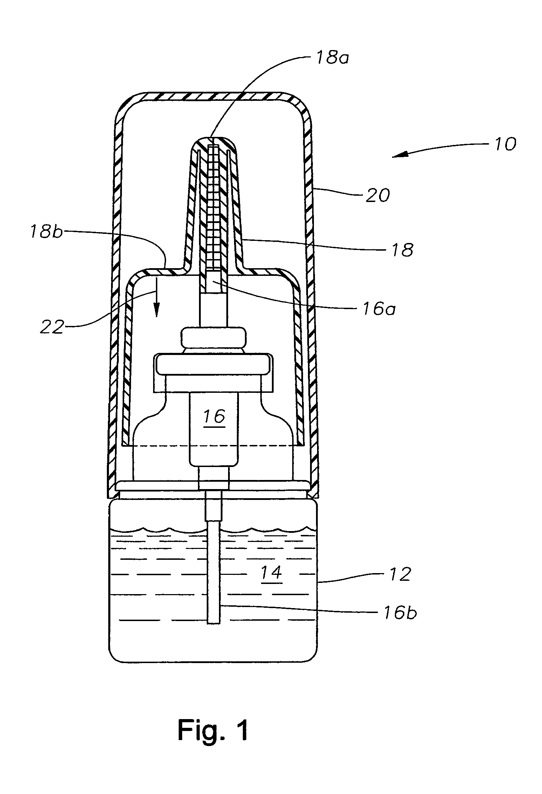

Figure 1 is a front, sectional view of a nasal sprayer according to a preferred embodiment of the present invention.

### Detailed Description of the Preferred Embodiments

The preferred embodiments of the present invention and their advantages are best understood by referring to Figure 1 of the drawings, like numerals being used for like and corresponding parts of the various drawings.

Unless indicated otherwise, all component amounts are presented on a % (w/v) basis and all references to olopatadine are to olopatadine free base.

Figure 1 shows a nasal sprayer 10 according to a preferred embodiment of the present invention. Nasal sprayer 10 generally includes a bottle 12 holding a formulation 14, a pump 16 sealingly engaged with bottle 12, an actuator 18 removably receiving a top portion 16a of pump 16, and a cap 20 removably engaged with bottle 12 and for covering actuator 18. Bottle 12 and cap 20 are preferably made from high-density polyethylene. A preferred pump for pump 16 is the Valois VP7/100S CS20 pump. Actuator 18 is preferably made from polypropylene. Formulation 14 is preferably an aqueous formulation that is effective for treating and/or preventing allergic or inflammatory conditions in the nose containing olopatadine. Formulation 14 preferably contains 0.38 - 0.62% olopatadine. Formulation 14 most preferably contains 0.6% olopatadine. After removing cap 20 and inserting nozzle 18a of actuator 18 into his or her nose, a user may deliver a single spray of formulation 14 from bottle 12 via tube 16b of pump 16 and actuator 18 by moving surface 18b of actuator 18 in the direction of arrow 22. Nasal sprayer 10 may be manufactured using conventional techniques.

It has been discovered that for a formulation 14 containing 0.6% olopatadine and a viscosity of 1 - 2 cps (preferably 1.7 cps), a certain spray characteristic emitted from nozzle 18a results in unexpectedly beneficial clinical performance. More specifically, a spray characteristic having one or more of the following parameters is preferred: a shot weight of 90 - 110 mg, and most preferably 100 mg; a spray pattern having a longest axis of 20 - 45 mm, and most preferably 23.5 mm; a spray pattern having a shortest axis of 14 - 20 mm, and most preferably 17.5 mm; a spray pattern having an ellipticity of 1 - 1.8, more preferably 1 - 1.4, and most preferably 1.24; and the following droplet size distribution:
D₁₀ = 15 - 30 µm, more preferably 18-25 µm, and most preferably 22 µm;
D₅₀ = 30 - 60 µm, more preferably 39-53 µm, and most preferably 47 µm;
D₉₀ = 50 - 150 µm, more preferably 83-128 µm, and most preferably 106 µm;
SPAN = not more than 3, more preferably 1.6 - 2.1, and most preferably 1.8;
% Volume < 10 µm = less than 4%, more preferably 0.8 - 2.4%, and most preferably 1.6%,
where D₁₀ is the droplet size distribution of 10% of the droplets, D₅₀ is the droplet size distribution of 50% of the droplets, D₉₀ is the droplet size distribution of 90% of the droplets; SPAN is the ratio of (D₉₀ - D₁₀) / D₅₀; and % Volume < 10 µm is the percentage of droplets less than 10 µm in diameter. D₁₀, D₅₀, and D₉₀ are measurements of the diameter of droplets. When two sprays/nostril of a formulation 14 containing 0.6% olopatadine having all the preferred or more-preferred parameters of the above-referenced spray characteristic were delivered twice per day in clinical trials involving over 4000 human subjects, pharmacokinetic testing revealed that this method of delivery produced a particularly advantageous bioavailability of olopatadine. More specifically, a peak plasma concentration of olopatadine (Cₘₐₓ) measured within 0.5 - 2 hours post-dose using high performance liquid chromatography from 14.4 - 35.3 ng/mL (mean 23.3 +/-6.1 ng/mL) was observed. This level of concentration is comparable to a concentration that would be expected to be obtained via a systemic (e.g. oral) dose form.

The following describes a preferred procedure for characterizing spray patterns. A TLC plate (e.g. silica gel 60, F₂₅₄ (florescence indicator), 250 µm thick layer on glass) and a TLC plate holder available from EM Science of Gibbstown, N.J.; a 254 nm filtered ultraviolet light source; and a camera suitable for taking pictures in ultraviolet light (e.g. a digital camera) are obtained. Sprayer 10 is loaded with formulation 14 and primed by actuating pump 16 via actuator 18 until a fine mist appears out of nozzle 18a. Sprayer 10 and the TLC plate holder are arranged so that nozzle 18a is about 3 cm from the TLC plate. Pump 16 is actuated via a conventional mechanical actuator using a constant force (preferably 5 kg). The resulting spray of formulation 14 is allowed to soak into the TLC plate. The TLC plate is moved to a dry section, and the procedure is repeated. For best results, two spray patterns are obtained from five separate units of sprayer 10. The patterns are viewed in 254 nm filtered ultraviolet light, and a photograph is taken of each pattern.

Using a printed photograph, each pattern is circled with a pencil. A single line is drawn to encircle all of the spray pattern, including any areas of density that appear to be apart from the rest of the pattern. The "outer ring", which is sometimes visible and is the result of liquid spreading out on the plate after contact, should not be circled. The inner, darker, pattern is the original spray pattern to be measured. Using a pencil, the longest axis that can be found within each circled pattern is drawn. The shortest axis that passes through the center of each longest axis is drawn. Each axis is measured to the nearest 0.5 mm. The ellipticity (shape) of each pattern is calculated according to the following: ellipticity = longest axis / shortest axis. The ellipticity is reported to the nearest 1/10. The longest axis, shortest axis, and ellipticity of each pattern for each sprayer 10 are averaged to provide one set of parameters for each sprayer 10. The parameters for all five units of sprayer 10 are then averaged to find a single set of spray pattern parameters for a given formulation 14.

The following describes a preferred procedure for characterizing droplet size distribution. Sprayer 10 is loaded with formulation 14 and primed by actuating pump 16 via actuator 18 until a fine mist appears out of nozzle 18a. Sprayer 10 and a commercially available laser diffraction instrument are arranged so that nozzle 18a is about 5 cm below the laser beam of the laser diffraction instrument. Pump 16 is actuated via a conventional mechanical actuator using a constant force (preferably 5 kg). The resulting spray of formulation 14 crosses the laser beam. Data are collected for D₁₀, D₅₀, D₉₀, SPAN, and % Volume < 10 µm. The average values for each of these parameters for two sprays are calculated.

From the above, it may be appreciated that the present invention provides improved methods of delivering topical olopatadine formulations that are effective for treating allergic or inflammatory conditions of the nose. It is believed that the operation and construction of the present invention will be apparent from the foregoing description.

## Claims

1. A nasal sprayer (10) having a bottle (12) having a formulation (14) comprising olopatadine, **characterised in** the sprayer being configured to deliver a spray of said formulation with a spray characteristic comprising at least one of:
a spray pattern having a longest axis of 20 - 45 mm, a shortest axis of 14 - 20 mm, and an ellipticity of 1 - 1.8, and/or
a droplet size distribution having a D₁₀ of 15 - 30 µm, a D₅₀ of 30 - 60 µm, a D₉₀ of 50 - 150 µm, a SPAN of not more than 3, and a % Volume of < 10 µm of less than 4%.

2. The sprayer of claim 1 having a spray characteristic wherein said longest axis is 23.5 mm.

3. The sprayer of claim 1 having a spray characteristic wherein said shortest axis is 17.5 mm.

4. The sprayer of claim 1 having a spray characteristic wherein said ellipticity is 1 - 1.4.

5. The sprayer of claim 1 wherein said formulation comprises 0.38 - 0.62 % olopatadine.

6. The sprayer of claim 5 wherein said formulation comprises 0.6% olopatadine.

7. The sprayer of claim 1 wherein said spray characteristic further comprises a shot weight of 90 -110 mg.

8. The sprayer of claim 1 having a droplet size distribution wherein D₁₀ is 18 - 25 µm, D₅₀ is 39 - 53 µm, D₉₀ is 83 - 128 µm, SPAN is 1.6 - 2.1, and % Volume of < 10 µm is 0.8 - 2.4%.

9. The sprayer of claim 1 having a droplet size distribution wherein: said droplet size distribution has a D₁₀ of 18 - 25 µm, a D₅₀ of 39 - 53 µm, a D₉₀ of 83 - 128 µm, a SPAN of 1.6 - 2.1, and a % Volume of < 10 µm of 0.8 - 2.4%; said formulation comprises 0.6% olopatadine; and said spray characteristic further comprises a shot weight of 90 - 110 mg.

10. Use of olopatadine in the manufacture of a nasal sprayer (10) for the treatment of allergic or inflammatory conditions of the nose comprising coupling a bottle (12) containing a formulation (14) of olopatadine to a nasal sprayer (10) and **characterised in** configuring the nasal sprayer to deliver a spray of said formulation with a spray characteristic comprising at least one of:
a spray pattern having a longest axis of 20 - 45 mm, a shortest axis of 14 - 20 mm, and an ellipticity of 1 - 1.8, and/or
a droplet size distribution having a D₁₀ of 15 - 30 µm, a D₅₀ of 30 - 60 µm, a D₉₀ of 50 - 150 µm, a SPAN of not more than 3, and a % Volume of < 10 µm of less than 4%.

11. Use as claimed in claim 10 wherein the olopatadine is provided as a formulation comprising 0.38 - 0.62 % olopatadine.

12. Use as claimed in claim 11 wherein said formulation comprises 0.6% olopatadine.

## Patentansprüche

1. Nasenspray-Zerstäuber (10), der eine Flasche (12) mit einer Formulierung (14) aufweist, die Olopatadin umfasst, **dadurch gekennzeichnet, dass** der Zerstäuber eingerichtet ist, um einen Sprühnebel dieser Formulierung mit einem Sprühcharakteristikum zuzuführen, das zumindest
ein Sprühmuster mit einer längsten Achse von 20 bis 45 mm, einer kürzesten Achse von 14 bis 20 mm und einer Elliptizität von 1 bis 1,8, und/oder
eine Tröpfchengrößenverteilung mit einem D₁₀ von 15 bis 30 µm, einem D₅₀ von 30 bis 60 µm, einem D₉₀ von 50 bis 150 µm, einem SPAN-Faktor von nicht mehr als 3 und einem %-Volumen von < 10 µm von weniger als 4 %
umfasst.

2. Zerstäuber nach Anspruch 1 mit einem Sprühcharakteristikum, wobei die längste Achse 23,5 mm ist.

3. Zerstäuber nach Anspruch 1 mit einem Sprühcharakteristikum, wobei die kürzeste Achse 17,5 ist.

4. Zerstäuber nach Anspruch 1 mit einem Sprühcharakteristikum, wobei die Elliptizität 1 bis 1,4 ist.

5. Zerstäuber nach Anspruch 1, wobei die Formulierung 0,38 % bis 0,62 % Olopatadin umfasst.

6. Zerstäuber nach Anspruch 5, wobei die Formulierung 0,6 % Olopatadin umfasst.

7. Zerstäuber nach Anspruch 1, wobei das Sprühcharakteristikum weiters ein Sprühgewicht von 90 bis 110 mg umfasst.

8. Zerstäuber nach Anspruch 1 mit einer Tröpfchengrößenverteilung, bei der D₁₀ 18 bis 25 µm, D₅₀ 39 bis 53 µm, D₉₀ 83 bis 128 µm, ein SPAN-Faktor 1,6 bis 2,1 und ein %-Volumen von < 10 µm von 0,8 bis 2,4 % ist.

9. Zerstäuber nach Anspruch 1 mit einer Tröpfchengrößenverteilung, wobei die Tröpfchengrößenverteilung einen D₁₀ von 18 bis 25 µm, D₅₀ von 39 bis 53 µm, D₉₀ von 83 bis 128 µm, einen SPAN-Faktor 1,6 bis 2,1 und ein %-Volumen von < 10 µm von 0,8 bis 2,4 % aufweist; wobei die Formulierung 0,6 % Olopatadin umfasst; und wobei das Sprühcharakteristikum weiters ein Sprühgewicht von 90 bis 110 mg umfasst.

10. Verwendung von Olopatadin bei der Herstellung eines Nasenspray-Zerstäubers (10) zur Behandlung von allergischen oder entzündlichen Zuständen der Nase, umfassend das Verbinden einer Flasche (12), die eine Formulierung (14) von Olopatadin enthält, mit einem Nasenspray-Zerstäuber (10), und **gekennzeichnet durch** Konfigurieren des Nasenspray-Zerstäubers zum Zuführen eines Sprühnebels der Formulierung mit einem Sprühcharakteristikum, das zumindest
ein Sprühmuster mit einer längsten Achse von 20 bis 45 mm, einer kürzesten Achse von 14 bis 20 mm und einer Elliptizität von 1 bis 1,8, und/oder
eine Tröpfchengrößenverteilung mit einem D₁₀ von 15 bis 30 µm, einem D₅₀ von 30 bis 60 µm, einem D₉₀ von 50 bis 150 µm, einem SPAN-Faktor von nicht mehr als 3 und einem %-Volumen von < 10 µm von weniger als 4 %
umfasst.

11. Verwendung nach Anspruch 10, wobei das Olopatadin als Formulierung vorgesehen ist, die 0,38 % bis 0,62 % Olopatadin umfasst.

12. Verwendung nach Anspruch 11, wobei die Formulierung 0,6 % Olopatadin umfasst.

## Revendications

1. Pulvérisateur nasal (10) ayant un flacon (12) ayant une formulation (14) comprenant de l'olopatadine, **caractérisé en ce que** le pulvérisateur est configuré pour administrer une pulvérisation de ladite formulation avec une caractéristique de pulvérisation comprenant au moins l'une parmi :
une forme de pulvérisation ayant un axe le plus long de 20 à 45 mm, un axe le plus court de 14 à 20 mm et une ellipticité de 1 à 1,8, et/ou
une distribution de taille de gouttelette ayant une D₁₀ de 15 à 30 µm, une valeur D₅₀ de 30 à 60 µm, une valeur D₉₀ de 50 à 150 µm, un intervalle de mesure non supérieur à 3 et un volume en % de moins de 10 µm inférieur à 4 %.

2. Pulvérisateur selon la revendication 1, ayant une caractéristique de pulvérisation dans laquelle ledit axe le plus long est de 23,5 mm.

3. Pulvérisateur selon la revendication 1, ayant une caractéristique de pulvérisation dans laquelle ledit axe le plus court est de 17,5 mm.

4. Pulvérisateur selon la revendication 1, ayant une caractéristique de pulvérisation dans laquelle ladite ellipticité est de 1 à 1,4.

5. Pulvérisateur selon la revendication 1, dans lequel ladite formulation comprend 0,38 à 0,62 % d'olopatadine.

6. Pulvérisateur selon la revendication 5, dans lequel ladite formulation comprend 0,6 % d'olopatadine.

7. Pulvérisateur selon la revendication 1, dans lequel ladite caractéristique de pulvérisation comprend en outre un poids injectable de 90 à 110 mg.

8. Pulvérisateur selon la revendication 1, ayant une distribution de taille de gouttelette dans laquelle D₁₀ est de 18 à 25 µm, D₅₀ est de 39 à 53 µm, D₉₀ est de 83 à 128 µm, l'intervalle de mesure est de 1, 6 à 2, 1 et le volume en % de moins de 10 µm est de 0,8 à 2,4 %.

9. Pulvérisateur selon la revendication 1, ayant une distribution de taille de gouttelette dans laquelle : ladite distribution de taille de gouttelette a une valeur D₁₀ de 18 à 25 µm, une valeur D₅₀ de 39 à 53 µm, une D₉₀ de 83 à 128 µm, un intervalle de mesure de 1,6 à 2,1 et un volume en % de moins de 10 µm de 0,8 à 2,4 % ; ladite formulation comprend 0,6 % d'olopatadine ; et ladite caractéristique de pulvérisation comprend en outre un poids injectable de 90 à 110 mg.

10. Utilisation d'olopatadine dans la fabrication d'un pulvérisateur nasal (10) destiné au traitement d'affections allergiques ou inflammatoires du nez comprenant le couplage d'un flacon (12) contenant une formulation (14) d'olopatatine à un pulvérisateur nasal (10) et **caractérisée par** la configuration du pulvérisateur nasal de sorte à administrer une pulvérisation de ladite formulation avec une caractéristique de pulvérisation comprenant au moins l'une parmi :
une forme de pulvérisation ayant un axe le plus long de 20 à 45 mm, un axe le plus court de 14 à 20 mm et une ellipticité de 1 à 1,8, et/ou
une distribution de taille de gouttelette ayant une valeur D₁₀ de 15 à 30 µm, une valeur D₅₀ de 30 à 60 µm, une valeur D₉₀ de 50 à 150 µm, un intervalle de mesure non supérieur à 3 et un volume en % de moins de 10 µm inférieur à 4 %.

11. Utilisation selon la revendication 10, dans laquelle de l'olopatadine est fournie sous la forme d'une formulation comprenant 0,38 à 0,62 % d'olopatadine.

12. Utilisation selon la revendication 11, dans laquelle ladite formulation comprend 0,6 % d'olopatadine.
